# EUROPEAN PATENT APPLICATION

(11) **EP 3 539 653 A1**
(43) Date of publication of application: **18.09.2019**
(21) Application number: 18382170.1
(22) Date of filing: 14.03.2018
(51) Int. Cl.: B01J 29/44, B01J 29/46, B01J 29/48, B01J 29/80, C07C 4/18, B01J 29/74, B01J 29/76, B01J 29/78, B01J 35/10, C07C 6/12

(54) **METHOD OF HEAVY REFORMATE CONVERSION INTO BTX OVER METAL-IMPREGNATED ZSM-5+SSZ-33 ZEOLITE COMPOSITE CATALYST**

(71) Applicant: Saudi Arabian Oil Company, Dhahran 31311 (SA); Instituto de Tecnologia Quimica (UPV-CSIC), 46022 Valencia (ES)
(72) Inventor: ABUDAWOUD, Raed Hasan, 31311 Dhahran (SA); AL-AMER, Mohammed I., 31311 Dhahran (SA); RUPNICKI, Leszek, 31311 Dhahran (SA); CORMA CANÓS, Avelino, 46022 Valencia (ES); PORTILLA OVEJERO, M. Teresa, 46022 Valencia (ES); MARTINEZ SÁNCHEZ, Cristina, 46022 Valencia (ES); AL-ZAHRANI, Ibrahim M., 31311 Dhahran (SA)
(74) Representative: Elzaburu S.L.P.

(57) **Abstract**

Methods of making BTX compounds including benzene, toluene, and xylene include feeding heavy reformate to a reactor containing a composite zeolite catalyst. The composite zeolite catalyst includes a mixture of SSZ-33 and ZSM-5. The SSZ-33, the ZSM-5, or both comprise one or more impregnated metals. The method further includes producing the BTX compounds by simultaneously performing transalkylation and dealkylation of the heavy reformate in the reactor. The composite zeolite catalyst is able to simultaneously catalyze both the transalkylation and dealkylation reactions.

## Description

### Technical Field

Embodiments of the present specification generally relate to methods of making aromatic compounds, and specifically relate to methods of making benzene, toluene, and xylenes from a heavy reformate feed.

### BACKGROUND

Heavy reformate (HR), containing mainly C₉₊ aromatics, is the fraction that remains after extraction of the more valuable BTX (benzene, toluene, xylenes) fraction from the catalytic reformate or the pyrolysis gasoline. Traditionally this fraction was directly added to the gasoline pool. However, due to the restriction of the benzene content in gasoline by environmental regulations, it is important to find alternative ways of upgrading this stream into other valuable products. One option is to convert the heavy aromatics in the heavy reformate into additional BTX compounds. Heavy reformate may be converted into xylenes, benzene, and toluene by dealkylation of the C₉₊ alkylaromatics to produce toluene, and further transalkylation of the toluene formed by dealkylation with other C₉₊ alkylaromatics present in the feed to produce benzene and xylenes. Regardless, these means to produce BTX compounds by simultaneous dealkylation and transalkylation have limited efficiency, because of the sequential nature of the conversion reaction process where products of a first reaction are utilized in a second reaction.

### SUMMARY

Accordingly, ongoing needs exist for catalysts suitable for efficiently converting heavy reformates to produce benzene, toluene, and xylenes. Embodiments of the present disclosure are related to composite zeolite catalyst formulations and methods of making benzene, toluene, and xylenes using the composite zeolite catalyst. The composite zeolite catalysts may convert a mixture of heavy aromatic compounds (such as those present in heavy reformate), particularly C₉₊ aromatic hydrocarbons to benzene, toluene, and xylenes, and particularly to commercially valuable xylenes.

According to one embodiment, a method of making BTX compounds including benzene, toluene, and xylene is provided. The method includes feeding heavy reformate to a reactor containing a composite zeolite catalyst. The composite zeolite catalyst includes a mixture of SSZ-33 and ZSM-5. The SSZ-33, the ZSM-5, or both comprise one or more impregnated metals. The method further includes producing the BTX compounds by simultaneously performing transalkylation and dealkylation of the heavy reformate in the reactor. The composite zeolite catalyst is able to simultaneously catalyze both the transalkylation and dealkylation reactions.

Additional features and advantages of the described embodiments will be set forth in the detailed description which follows, and in part will be readily apparent to those skilled in the art from that description or recognized by practicing the described embodiments, including the detailed description which follows, the claims, as well as the appended drawings.

### BRIEF DESCRIPTION OF THE DRAWINGS

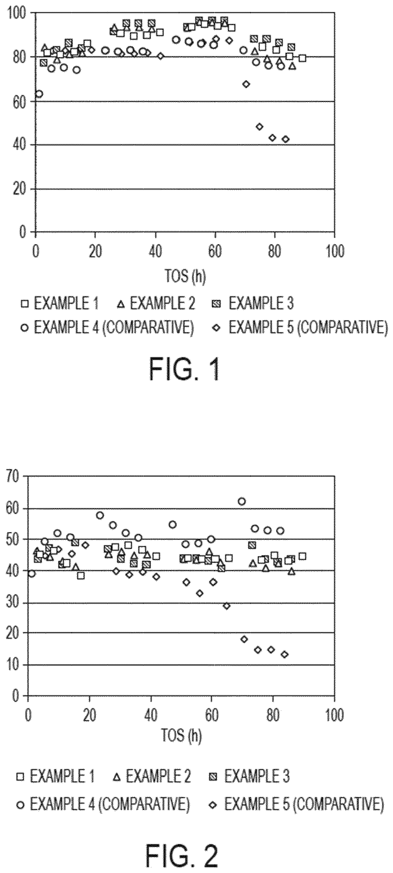

FIG. 1 is a graph of Methylethylbenzene (MEB) conversion of a simulated heavy reformate stream obtained with composite zeolite catalysts containing SSZ-33 and a commercially available ZSM-5 in accordance with one or more embodiments of the present disclosure (Examples 1, 2 and 3), SSZ-33 (Example 4, comparative) and a commercial multizeolite catalyst (Example 5, comparative).
FIG. 2 is a graph of Trimethylbenzene (TMB) conversion of a simulated heavy reformate stream obtained with composite zeolite catalysts containing SSZ-33 and a commercially available ZSM-5 in accordance with one or more embodiments of the present disclosure (Examples 1, 2 and 3), SSZ-33 (Example 4, comparative) and a commercial multizeolite catalyst (Example 5, comparative).

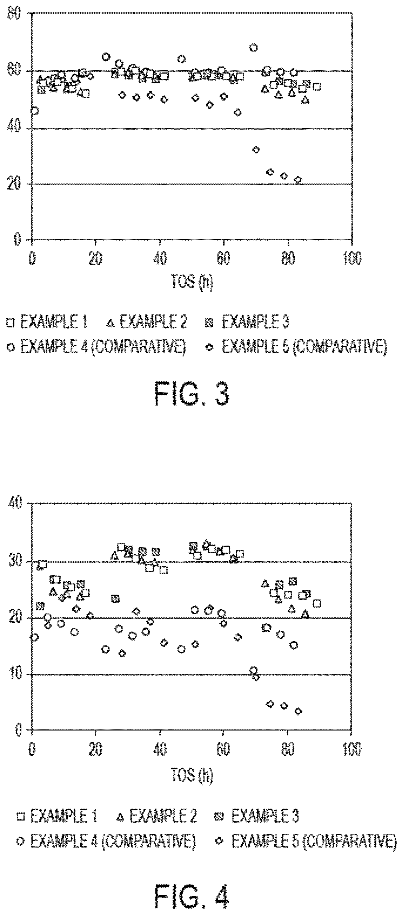

FIG. 3 is a graph of overall conversion (MEB+TMB) of a simulated heavy reformate stream obtained with composite zeolite catalysts containing SSZ-33 and a commercially available ZSM-5 in accordance with one or more embodiments of the present disclosure (Examples 1, 2 and 3), SSZ-33 (Example 4, comparative) and a commercial multizeolite catalyst (Example 5, comparative).
FIG. 4 is a graph of xylenes yield from a simulated heavy reformate stream obtained with composite zeolite catalysts containing SSZ-33 and a commercially available ZSM-5 in accordance with one or more embodiments of the present disclosure (Examples 1, 2 and 3), SSZ-33 (Example 4, comparative) and a commercial multizeolite catalyst (Example 5, comparative).

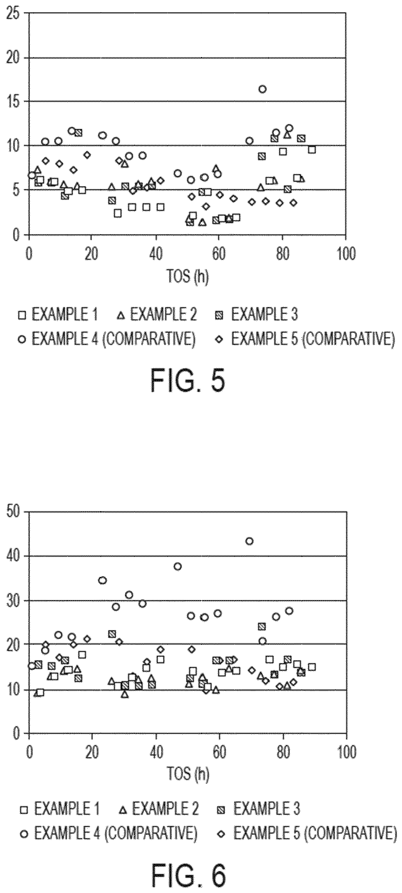

FIG. 5 is a graph of A₁₀ yield (yield of aromatics with 10 carbons) from a simulated heavy reformate stream obtained with composite zeolite catalysts containing SSZ-33 and a commercially available ZSM-5 in accordance with one or more embodiments of the present disclosure (Examples 1, 2 and 3), SSZ-33 (Example 4, comparative) and a commercial multizeolite catalyst (Example 5, comparative).
FIG. 6 is a graph of A₁₀₊ yield (yield of aromatics with more than 10 carbons) from a simulated heavy reformate stream obtained with composite zeolite catalysts containing SSZ-33 and a commercially available ZSM-5 in accordance with one or more embodiments of the present disclosure (Examples 1, 2 and 3), SSZ-33 (Example 4, comparative) and a commercial multizeolite catalyst (Example 5, comparative).

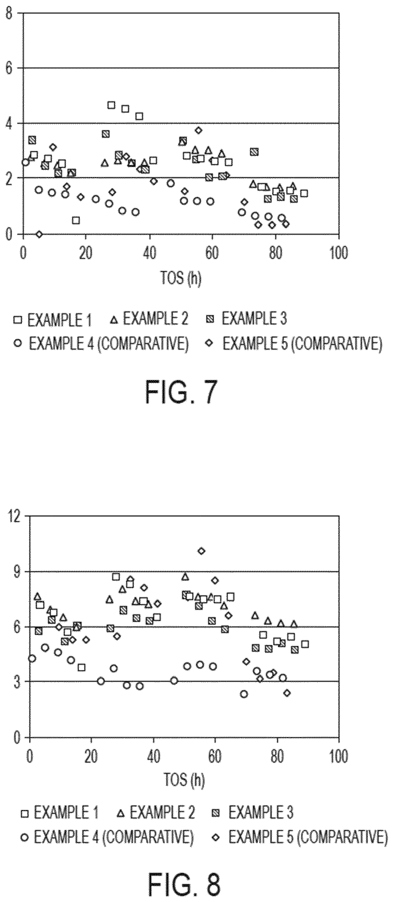

FIG. 7 is a graph of light hydrocarbon yield from a simulated heavy reformate stream obtained with composite zeolite catalysts containing SSZ-33 and a commercially available ZSM-5 in accordance with one or more embodiments of the present disclosure (Examples 1, 2 and 3), SSZ-33 (Example 4, comparative) and a commercial multizeolite catalyst (Example 5, comparative).
FIG. 8 is a graph of toluene yield from of a simulated heavy reformate stream obtained with composite zeolite catalysts containing SSZ-33 and a commercially available ZSM-5 in accordance with one or more embodiments of the present disclosure (Examples 1, 2 and 3), SSZ-33 (Example 4, comparative) and a commercial multizeolite catalyst (Example 5, comparative).

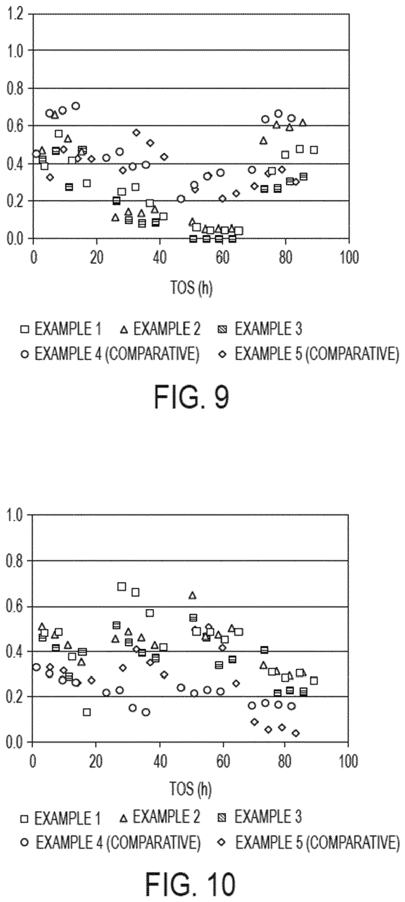

FIG. 9 is a graph of ethylbenzene yield from a simulated heavy reformate stream obtained with composite zeolite catalysts containing SSZ-33 and a commercially available ZSM-5 in accordance with one or more embodiments of the present disclosure (Examples 1, 2 and 3), SSZ-33 (Example 4, comparative) and a commercial multizeolite catalyst (Example 5, comparative).
FIG. 10 is a graph of benzene yield from a simulated heavy reformate stream obtained with composite zeolite catalysts containing SSZ-33 and a commercially available ZSM-5 in accordance with one or more embodiments of the present disclosure (Examples 1, 2 and 3), SSZ-33 (Example 4, comparative) and a commercial multizeolite catalyst (Example 5, comparative).

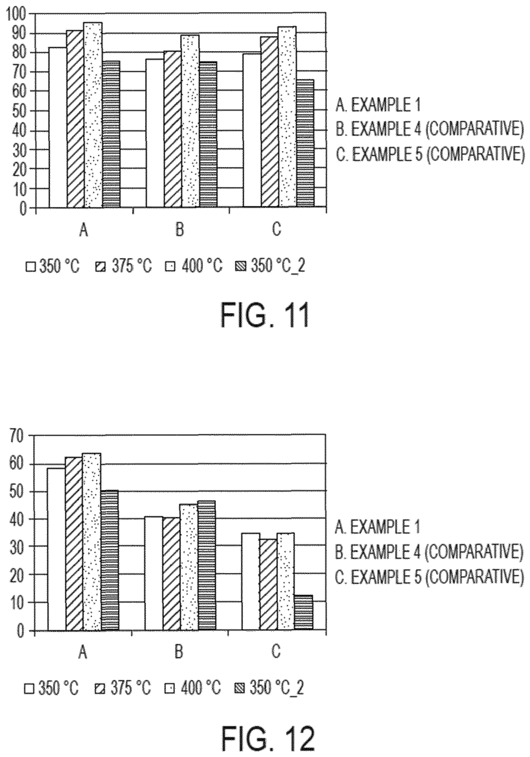

FIG. 11 is a graph of MEB conversion of an industrial heavy reformate stream obtained with a composite zeolite catalyst containing SSZ-33 and a commercially available ZSM-5 in accordance with one or more embodiments of the present disclosure (Example 1), SSZ-33 (Example 4) and a commercial multizeolite catalyst (Example 5).
FIG. 12 is a graph of TMB conversion of an industrial heavy reformate stream obtained with a composite zeolite catalyst containing SSZ-33 and a commercially available ZSM-5 in accordance with one or more embodiments of the present disclosure (Example 1), SSZ-33 (Example 4) and a commercial multizeolite catalyst (Example 5).

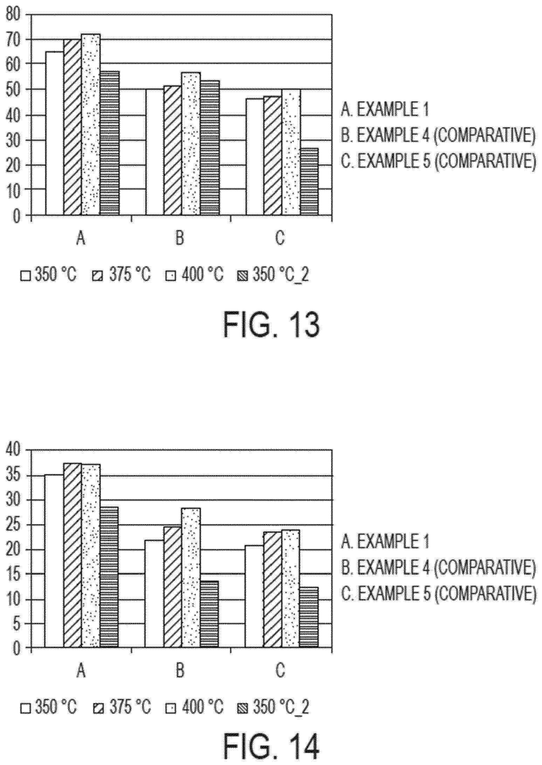

FIG. 13 is a graph of overall conversion of an industrial heavy reformate stream obtained with a composite zeolite catalyst containing SSZ-33 and a commercially available ZSM-5 in accordance with one or more embodiments of the present disclosure (Example 1), SSZ-33 (Example 4) and a commercial multizeolite catalyst (Example 5).
FIG. 14 is a graph of xylenes yield from an industrial heavy reformate stream obtained with a composite zeolite catalyst containing SSZ-33 and a commercially available ZSM-5 in accordance with one or more embodiments of the present disclosure (Example 1), SSZ-33 (Example 4) and a commercial multizeolite catalyst (Example 5).

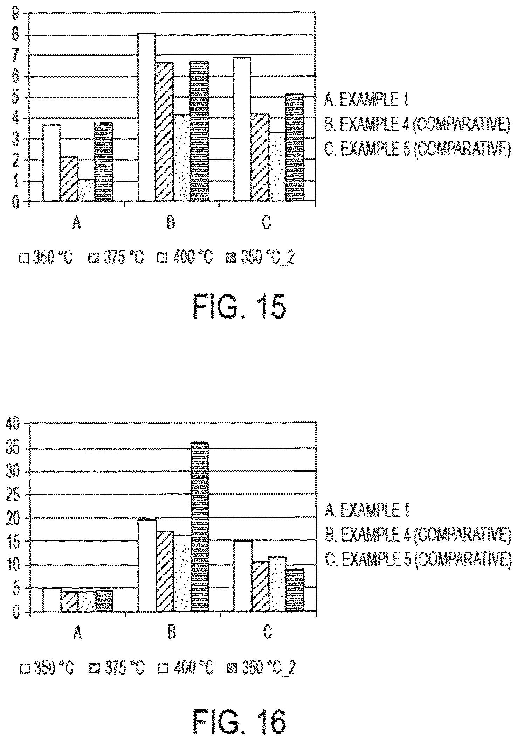

FIG. 15 is a graph of A₁₀ yield from an industrial heavy reformate stream obtained with a composite zeolite catalyst containing SSZ-33 and a commercially available ZSM-5 in accordance with one or more embodiments of the present disclosure (Example 1), SSZ-33 (Example 4) and a commercial multizeolite catalyst (Example 5).
FIG. 16 is a graph of A₁₀₊ yield from an industrial heavy reformate stream obtained with a composite zeolite catalyst containing SSZ-33 and a commercially available ZSM-5 in accordance with one or more embodiments of the present disclosure (Example 1), SSZ-33 (Example 4) and a commercial multizeolite catalyst (Example 5).

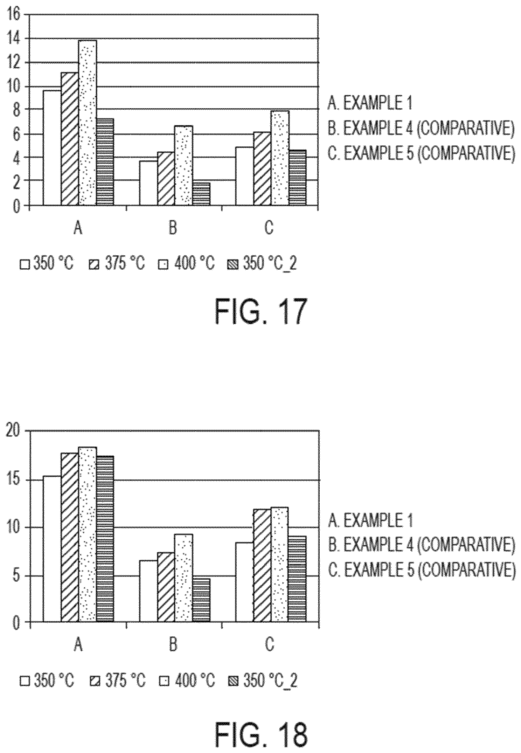

FIG. 17 is a graph of light hydrocarbon yield from an industrial heavy reformate stream obtained with a composite zeolite catalyst containing SSZ-33 and a commercially available ZSM-5 in accordance with one or more embodiments of the present disclosure (Example 1), SSZ-33 (Example 4) and a commercial multizeolite catalyst (Example 5).
FIG. 18 is a graph of toluene yield from of an industrial heavy reformate stream obtained with a composite zeolite catalyst containing SSZ-33 and a commercially available ZSM-5 in accordance with one or more embodiments of the present disclosure (Example 1), SSZ-33 (Example 4) and a commercial multizeolite catalyst (Example 5).

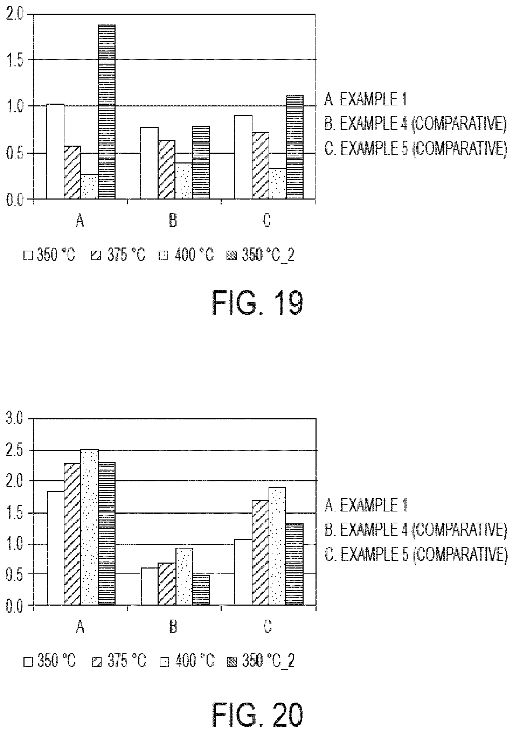

FIG. 19 is a graph of ethylbenzene yield from an industrial heavy reformate stream obtained with a composite zeolite catalyst containing SSZ-33 and a commercially available ZSM-5 in accordance with one or more embodiments of the present disclosure (Example 1), SSZ-33 (Example 4) and a commercial multizeolite catalyst (Example 5).
FIG. 20 is a graph of benzene yield from an industrial heavy reformate stream obtained with a composite zeolite catalyst containing SSZ-33 and a commercially available ZSM-5 in accordance with one or more embodiments of the present disclosure (Example 1), SSZ-33 (Example 4) and a commercial multizeolite catalyst (Example 5).

### DETAILED DESCRIPTION

Reference will now be made in detail to embodiments of a method of making benzene, toluene, and xylene by conversion of heavy reformate with a composite zeolite catalyst.

The main components of heavy reformate are ethyl-toluenes (methyl-ethyl-benzenes, MEB) and trimethyl-benzenes (TMB). The structures of the MEB isomers and TMB isomers are provided *infra.*

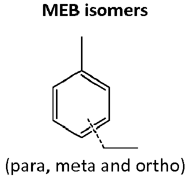

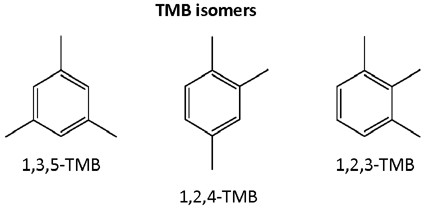

These aromatics can be converted into the more valuable BTX compounds by means of dealkylation of the C₉₊ alkylaromatics, or by transalkylation of these compounds with benzene or toluene. The aim of the process is to maximize the production of xylenes by de-ethylation of MEB and transalkylation of TMB. Specifically, transalkylation of TMB present in the feed with the toluene formed as a product of de-ethylation of MEB.

The dealkylation of MEB to toluene and ethane is provided *infra.* Dealkylation of MEB in the presence of a Brønsted acid catalyst initially produces toluene and ethylene. However, the ethylene may be subsequently hydrogenated to ethane in the presence of an adequate hydrogenation catalyst. If the hydrogenation functionality is not effective, portions of the ethylene may not be hydrogenated to ethane and as such may be present in the product gases, or it may be converted to oligomers or other products.

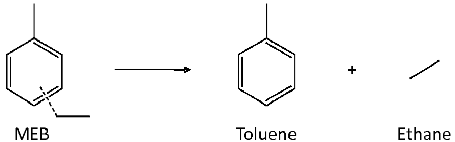

The transalkylation of TMB present in the heavy reformate with the toluene formed from dealkylation of MEB to toluene and ethane is provided *infra.*

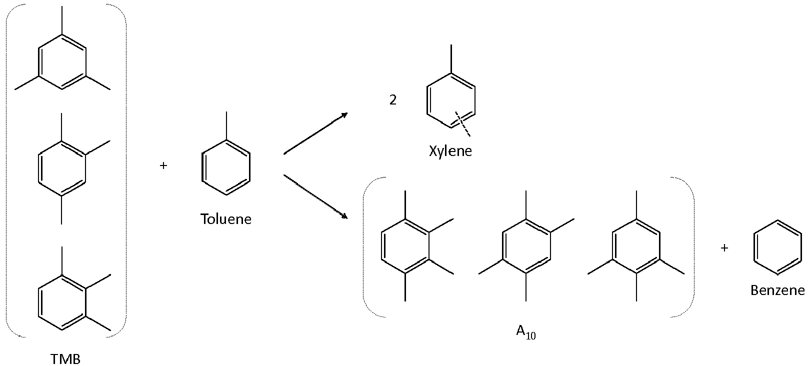

Additionally, toluene and TMB may also undergo a disproportionation reaction leading to xylenes and benzene or xylenes and tetramethylbenzenes (A₁₀), respectively. The chemical reactions are provided *infra.*

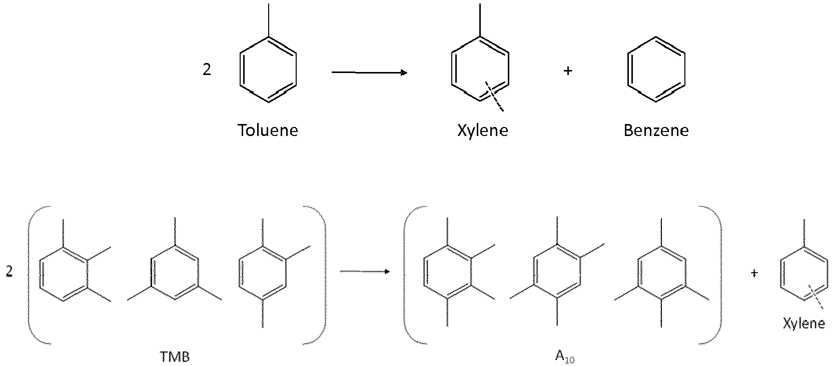

A composite zeolite catalyst may be formed from a mixture of SSZ-33 and ZSM-5 zeolite catalysts. The composite zeolite catalyst allows conversion of heavy reformate, or other aromatic reactant streams, in a single reactor. Specifically, the dealkylation of MEB and the transalkylation of the produced toluene with TMB may be performed in a single reactor, because of the proximity between the crystals of SSZ-33 and ZSM-5 when physically mixed in a single reactor. The MEB dealkylation reaction is necessary in order to obtain the toluene that has to react with the TMB in the feed for producing the desired xylenes. Thus, the proximity of the ZSM-5 and SSZ-33 crystals obtained by the physical mixing of the SSZ-33 and ZSM-5 and reaction in a single reactor enables an improved and faster coupling of both consecutive reactions to produce benzene, toluene, and xylenes.

The method of making BTX compounds includes feeding heavy reformate to a reactor which contains the composite zeolite catalyst formed from a mixture of SSZ-33 and ZSM-5. The BTX compounds are then produced by simultaneously performing transalkylation and dealkylation of the heavy reformate in the reactor. The composite zeolite catalyst is able to simultaneously catalyze both the transalkylation and dealkylation reaction with the combination of SSZ-33 and ZSM-5.

The composite zeolite catalyst including both SSZ-33 and ZSM-5 produces a synergistic effect and is capable of converting a heavy reformate feed to BTX compounds within a single reactor. The presence of ZSM-5 component increases the amount of toluene in the reaction media as a result of the additional dealkylation activity originating from the ZSM-5 component. The increased availability of toluene in the reaction media allows for increased reaction with the TMB present in the heavy reformate feed in conjunction with the SSZ-33 to yield BTX products, especially desirable xylenes. Additionally, as the TMB has more toluene available for reaction, transalkylation of the A₉₊ aromatics from the heavy reformate feed is reduced, which also reduces the generation of undesirable A₁₀₊ by-products.

Alkylaromatics, such as those present in a heavy reformate (MEB, TMB), in the presence of an acid catalyst, may undergo undesired reactions, which lead to formation of aromatics with more than 10 carbon atoms (A₁₀₊). If these A₁₀₊ compounds cannot diffuse out of the zeolite crystals through the pores of the crystalline structure because of steric limitations, they may block part of the channel systems or lead to bulkier coke precursors. The improved conversion efficiency of the composite zeolite catalysts alleviates the formation of heavy alkylaromatics (A₁₀ and A₁₀₊). Specifically, the proximity of the ZSM-5 and SSZ-33 as a mixture within a single reactor allows the TMB of the feed to react preferentially with the toluene formed by dealkylation of MEB on the ZSM-5 crystals, instead of reacting with other TMB by transalkylation to form tetramethylbenzene or heavier compounds. The reaction of the heavy reformate feed in a single reactor in the presence of the composite zeolite catalyst, including ZSM-5 and SSZ-33, results in greater selectivity to xylenes and reduced formation of A₁₀₊ and coke precursors, leading therefore to improved catalyst life.

The reaction of the heavy reformate feed in a single reactor with the composite zeolite catalyst comprising ZSM-5 and SSZ-33 achieves improved performance in conversion of the heavy reformate to BTX compounds. This improvement is even more profound when carrying out the transalkylation of a heavy reformate in the absence of added toluene or benzene, because these two aromatics must be produced *in-situ* from C₉₊ aromatics such as with dealkylation of MEB contained within the feed. The proximate locations of the SSZ-33 and ZSM-5 in the composite zeolite catalyst through a physical mixture and reaction in a single reactor allows the toluene produced from dealkylation of MEB to be more readily available for use in the transalkylation reaction of TMB or disproportionation reaction of toluene for the ultimate production of xylenes.

The composite zeolite catalyst in one or more embodiments comprises SSZ-33 and ZSM-5. ZSM-5 is an aluminosilicate zeolite of the pentasil family of zeolites. ZSM-5 (Zeolite Socony Mobil-5) has a Mordenite Framework Inverted (MFI) framework with an ordered crystal structure presenting intersecting 10-ring channels (5.1x5.5 and 5.3x5.6 angstrom (Å)). SSZ-33 is an aluminosilicate with a CON-type framework presenting intersecting 10-ring (4.5x5.1 Å) and 12-ring pores (6.4 x7.0, 5.9x7.0 Å). SSZ-33 may be obtained as a borosilicate, Boron-SSZ-33 (B-SSZ-33), and as an aluminosilicate, Aluminum-SSZ-33 (Al-SSZ-33). In one or more embodiments, the SSZ-33 in the composite zeolite catalyst is an Al-SSZ-33. B-SSZ-33 and Al-SSZ-33 both present a crystalline structure with Si⁴⁺ in a tetrahedral position connected by oxygen bridges to other atoms in tetrahedral positions. A negative charge appears in the framework which must be compensated for by a cation when Si⁴⁺ is substituted with Al³⁺ or B³⁺. The introduction of a proton forms a Brønsted acid site which is the active site of an acid zeolite. Without wishing to be bound by theory, the Brønsted acid sites generated by the presence of B are less strong than those generated by the presence of Al and thus, in embodiments aluminosilicate is preferred. However, initial generation of borosilicate is easier due to thermodynamic considerations than aluminosilicate such that borosilicate is initially formed and the final active zeolite, Al-SSZ-33, is generated through additional conversion steps.

The large pore SSZ-33 and medium pore ZSM-5 zeolite catalysts may be physically mixed in various ratios to produce a composite zeolite catalyst with varying degrees of MEB and TMB conversion. In one or more embodiments, the SSZ-33 and ZSM-5 are combined in a 50:50 to 90:10 weight ratio to form the composite zeolite catalyst. In various further embodiments, the SSZ-33 and ZSM-5 are combined in a 50:50 to 80:20 weight ratio, 50:50 to 70:30 weight ratio, 55:45 to 65:35 weight ratio, or an approximately 60:40 weight ratio to form the composite zeolite catalyst. As previously indicated, an increase in ZSM-5 in the composite zeolite catalyst results in an increase in the amount of toluene in the reaction media as a result of the additional dealkylation activity originated from the ZSM-5 component. However, it will be appreciated that an increase in ZSM-5 weight percentage (wt. %) requires a congruent reduction in SSZ-33 wt. %. Reduction in SSZ-33 wt. % would result in less transalkylation and an excess of toluene formation. Transalkylation reactions need larger micropore volume to proceed than dealkylation because of the involvement of two alkylaromatic molecules. The structure of the SSZ-33 with the 10-ring and 12-ring channels is more conducive to transalkylation than ZSM-5 with only 10-ring pores.

Moreover, the zeolite composite catalyst may be impregnated with metals for catalysis, for example, metals such as molybdenum, chromium, platinum, nickel, tungsten, palladium, ruthenium, gold, rhenium, rhodium, or combinations thereof. In one embodiment, the impregnated metal is rhenium (Re). The metal component may exist within the final zeolite composite catalyst as a compound, such as an active metal oxide, an active metal sulfide or active metal halide, in chemical combination with one or more of the other ingredients of the composite, or as an elemental metal. The impregnated metal component may be present in the final composite zeolite catalyst in any amount that is catalytically effective, for example from 0.01 to 20.0 wt. %, or from 2 to 5 wt. %, or from 0.1 to 1.5 wt. %, or approximately 0.5 wt. % of the composite zeolite catalyst particle.

In various embodiments, the impregnated metal component may be present in only the SSZ-33, only the ZSM-5, or both. For example, the impregnated metal component may be in the SSZ-33 or ZSM-5 individually from 0.01 to 20.0 wt. %, or from 2 to 5 wt. %, or from 0.1 to 1.5 wt. %, or from 0.1 to 0.5 wt. %, or from 0.25 to 0.55 wt. %, or approximately 0.3 wt. % of the SSZ-33 or ZSM-5.

Metals are added to the catalyst for their hydrogenation functionality. The dealkylation, transalkylation and disproportionation reactions take place on the Brønsted acid sites of the composite zeolite catalysts. However, the hydrogenation function of the metal component is utilized to convert ethylene into ethane and may also enhance the desorption of coke precursors. The conversion of ethylene into ethane avoids the oligomerization of the olefin to products that may deactivate the catalyst.

In one or more embodiments, the metals are incorporated into the zeolite composite catalyst by ion exchange or impregnation of their salts in aqueous solution. The catalysts with the incorporated metals are then calcined in air and the metals are converted into their oxide forms, which do not present hydrogenation activity. In order to be active for hydrogenation, these oxides are converted into metal sulfides, for example metal sulfides of Mo, Ni, or W, or the metal oxides can be reduced to their elemental metal form, for example elemental forms of Mo, Pt, Re, Pd, or Rh. In one or more embodiments, the composite zeolite catalyst is impregnated with rhenium in the form of ammonium perrhenate (NH₄ReO₄) through an incipient wetness procedure. In one or more embodiments, the composite zeolite catalyst is impregnated with molybdenum in the form of ammonium molybdate tetrahydrate ((NH₄)₆Mo₇O₂₄ · 4H₂O) through an incipient wetness procedure.

In one embodiment, the molar ratio of silicon to aluminum (Si/Al) in the SSZ-33 zeolite present in the composite catalyst is from 5:1 to 50:1. In further embodiments, the molar ratio of silicon to aluminum in the SSZ-33 zeolite present in the composite catalyst is from 8:1 to 25:1 or from 10:1 to 15:1. In one embodiment, the molar ratio of silicon to aluminum (Si/Al) in the ZSM-5 zeolite present in the composite catalyst is from 5:1 to 200:1. In further embodiments, the molar ratio of silicon to aluminum in the ZSM-5 zeolite present in the composite catalyst is from 8:1 to 100:1, from 10:1 to 50:1, or from 10:1 to 25:1. In various embodiments, the SSZ-33 and ZSM-5 may each individually have molar ratios of silicon to aluminum of from 5:1 to 100:1, from 8:1 to 25:1, or from 10:1 to 15:1.

In one or more embodiments, the ZSM-5 zeolite catalyst is a commercially available ZSM-5. For example, the ZSM-5 may be CBV3024E from Zeolyst International (Conshohocken, Pennsylvania, USA).

SSZ-33 is not a commercially available zeolite catalyst and therefore must be synthesized for utilization in the composite zeolite catalyst. It will be appreciated that various synthesis procedures may be utilized to prepare the SSZ-33 for utilization in the composite zeolite catalyst. One example synthesis procedure is detailed in the Examples section of this disclosure.

### EXAMPLES

The described embodiments will be further clarified by the following examples and comparative examples.

The following synthesis procedure for SSZ-33 serves as an example and differing synthesis procedures may equally be utilized to prepare the SSZ-33 for utilization in the composite zeolite catalyst. Synthesis of SSZ-33 includes synthesis of an organic structure directing agent (OSDA) and synthesis of Boron-Beta (B-Beta) as precursors to the final synthesis of the SSZ-33.

The organic structure directing agent allows for the crystallization of the SSZ-33 in the desired configuration. The organic structure directing agent is provided as a hydroxide cation as illustrated by Structure [1].

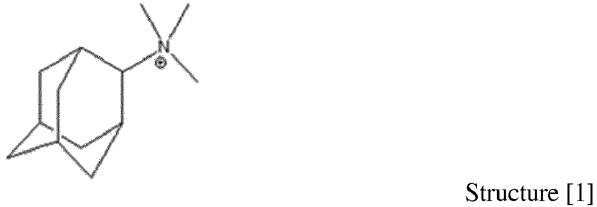

The OSDA cation for the SSZ-33 synthesis (Structure [1]) may be prepared by dissolving 24.9 grams (g) of 2-adamantylamine hydroxide (Sigma-Aldrich, 98 wt. %) in 250 milliliters (ml) of methanol. To the resulting mixture, 50 g of potassium carbonate (Sigma-Aldrich, 99 wt. %) is added and the mixture is ice cooled. Further, 62 g of methyl iodide is added dropwise and the resultant mixture is kept under agitation for 4 days at room temperature. The resulting precipitate is filtered and washed with diethyl ether with the resultant solid extracted with chloroform using a Soxhlet extractor, for example, as an organic salt. The iodide form of the formed organic salt is exchanged to the hydroxide form by dissolving 16 g of the OSDA in the iodide form (OSDA(I)) in water and adding 50 g of Dowex® SBR anion exchange resin (Dow Chemical Company). The resulting mixture is maintained under agitation overnight and filtered to obtain the hydroxide form of the OSDA cation (OSDA(OH) - Structure [1]).

B-Beta is synthesized as a silicon source for preparation of the SSZ-33 zeolite. The B-Beta may be prepared by forming a first solution of 10.4 g of distilled water, 7.35 g of tetraethylammonium hydroxide (TEAOH, Sigma-Aldrich, 35 wt. %), and 1.55 g of boric acid (Sigma-Aldrich 98 wt. %) and mixing for 20 minutes. A second solution of 10 g of distilled water, 45.36 g of tetraethylammonium hydroxide, and 15 g of fumed silica (AEROSIL® 200, Evonik Corp., Parsippany, NJ, USA) is formed and mixed for 20 minutes. The first solution is then added over the second solution and the resultant mixture is stirred for 30 minutes. The resulting gel is transferred to an autoclave with a polytetrafluoroethylene liner (commonly known as Teflon®), and heated at 150°C for 4 days under dynamic conditions by agitation of the gel in the autoclave. The resulting powder after filtration and washing with distilled water is B-Beta. The as-prepared B-Beta is then further calcined at 580°C in air for 6 hours to remove any occluded organic molecules in preparation for SSZ-33 synthesis.

To synthesize SSZ-33 for utilization in the composite zeolite catalyst, Boron-SSZ-33 (B-SSZ-33) is initially synthesized. The B-SSZ-33 zeolite is prepared by mixing 14.94 g of distilled water with 10.53 g of an aqueous solution of OSDA(OH) (Structure [1]) (9.5 wt. %) and 0.70 g of an aqueous solution of sodium hydroxide (20 wt. %). To the resulting mixture 0.187 g of anhydrous sodium tetraborate (Sigma-Aldrich, 98 wt. %) and 2 g of B-Beta are added. The resulting gel is stirred for 30 minutes. The molar composition of the gel is 1 SiO₂ : 0.105 B₂O₃ : 0.15 OSDA(OH) : 0.17 NaOH : 44 H₂O. The gel is transferred to an autoclave with a polytetrafluoroethylene liner and heated at 150°C for 10 days under static conditions. The resulting powder after filtration and washing with distilled water is B-SSZ-33. The as-prepared B-SSZ-33 is then calcined at 580°C in air for 6 hours to remove any occluded organic molecules.

To synthesize SSZ-33 for utilization in the composite zeolite catalyst, generally the Al-SSZ-33 form, the B-SSZ-33 is converted to Al-SSZ-33. To prepare Al-SSZ-33 an isomorphic substitution of B for Al atoms is required. The Al-SSZ-33 is prepared by mixing 7.3 g of the calcined B-SSZ-33 zeolite with 365 g of an aqueous solution of aluminum nitrate (Sigma-Aldrich, 5 wt. %) and keeping the resulting solution under reflux conditions for 48 hours. The resulting solid is filtered and washed with abundant distilled water and then dried at 100 C overnight. The dried powder is the Al-SSZ-33 for utilization in the composite zeolite catalyst.

For demonstration purposes, composite zeolite catalysts were prepared in accordance with one or more embodiments of this disclosure. Additionally, comparative samples of pure Al-SSZ-33 were synthesized in accordance with the method previously disclosed. The composite zeolite catalysts were formed as a physical mixture of Al-SSZ-33 and ZSM-5. The Al-SSZ-33 and ZSM-5 were mixed in a 40:60 weight ratio of SSZ-33:ZSM-5, a 60:40 weight ratio of SSZ-33:ZSM-5, and a 80:20 weight ratio of SSZ-33:ZSM-5. The ZSM-5 was the commercially available CBV3024E (Zeolyst International). The ZSM-5 zeolite catalyst and the Al-SSZ-33 catalyst were impregnated with rhenium before being mixed in order to prepare the 40:60, 60:40, and 80:20 SSZ-33:ZSM-5 composite zeolite catalyst. Rhenium was incorporated into each zeolite at 0.3 wt. % by means of the incipient wetness procedure using ammonium perrhenate (NH₄ReO₄) as a metal precursor. After rhenium loading, the zeolites were stored in a desiccator for at least 5 hours and then dried at 100°C overnight. After mixing the rhenium containing zeolites the SSZ-33:ZSM-5 composite zeolite catalysts were calcined in a fixed bed reactor with the temperature increased up to 500°C in a flow of nitrogen gas (N₂) at 100 milliliter per minute (ml/min) and then maintained at 500°C for 3 hours under air flow at 100 ml/min. The samples were then cooled to room temperature under a nitrogen flow at 100 ml/min. The prepared composite zeolite catalyst with a 60:40 weight ratio of SSZ-33:ZSM-5 is designated as Example 1, the prepared composite zeolite catalyst with a 40:60 weight ratio of SSZ-33:ZSM-5 is designated as Example 2, the prepared composite zeolite catalyst with a 80:20 weight ratio of SSZ-33:ZSM-5 is designated as Example 3, and the pure SSZ-33 is designated as Example 4 (comparative). Additionally, a commercially available AC-140 sample was considered. AC-140 is a commercially available multizeolite catalyst which is formed from PtNi/Beta (60 wt. %), ZSM-5 (20 wt. %), and Ce/AP-3 (20 wt. %). The AC-140 sample is designated as Example 5 (comparative). Further, samples of commercially available ZSM-5 (CBV3024E from Zeolyst International) impregnated with rhenium at 0.3 wt. % were prepared and designated Example 6 (comparative).

The physico-chemical properties of each of zeolite catalysts were quantified. Specifically, the silicon to aluminum ratio as well as the final wt. % of Re in each sample was determined for each catalyst type. Additionally, the micropore volume and the mesopore volume were calculated according to the t-plot method and the BJH correlation method respectively. Further, the micropore surface area was calculated from the micropore volume and the total specific surface area was calculated in accordance with the Brunauer-Emmett-Teller (BET) method widely used for evaluating the surface area of porous and finely-divided materials. These physio-chemical properties are delineated in Table 1 provide *infra.*

**Table 1: Chemical composition and textural properties of samples**

| **Sample** | **Si/Al** | **Re (wt.%)** | **S_{BET} (m²/g)** | **S _{micro} (m²/g)** | **V_{micro} (cm³/g)** | **Vₘₑₛₒ (cm³/g)** |
|---|---|---|---|---|---|---|
| SSZ-33 | 12.2 | --- | 514 | 475 | 0.233 | 0.05 |
| Re/SSZ-33 Example 4 (comparative) | 12.6 | 0.3 | 503 | 465 | 0.228 | 0.05 |
| ZSM-5 (CBV3024E) | 13.0 | --- | 372 | 332 | 0.162 | 0.055 |
| Re/ZSM-5 Example 6 (comparative) | 13.4 | 0.3 | 365 | 327 | 0.159 | 0.054 |
| AC-140 Example 5 (comparative) | 2.9 | --- | 457 | 372 | 0.18 | 0.26 |

Table 1 illustrates the property differences between SSZ-33, ZSM-5, and the commercially available AC-140 multizeolite catalyst. It is noted that SSZ-33 and ZSM-5 present similar bulk Si/Al ratios and the ratios remain similar upon impregnation with rhenium. Additionally, SSZ-33 presents larger S_{BET}, S_{micro}, and V_{micro} than the ZSM-5 samples, these differences are believed the result of the combination of 10-ring and 12-ring channels in SSZ-33 and only 10-ring pores in ZSM-5. The combination of SSZ-33 and ZSM-5 in the composite zeolite catalyst allows for the differing properties of the SSZ-33 and ZSM-5 to work in a synergistic manner to provide improved dealkylation and transalkylation of the heavy reformate feed.

The acidic properties of each of the zeolite catalysts were also quantified. Acidity measurements were carried out by adsorption/desorption of pyridine followed by IR spectroscopy. Self-supported wafers (10 milligrams per square centimeter (mg cm⁻²)) of calcined samples, previously activated at 400°C and 10⁻² Pascal (Pa) overnight in a Pyrex vacuum cell, were allowed to come in contact with 6.5 × 10² Pa of pyridine vapor at room temperature and desorbed in vacuum at increasing temperatures (150°C, 250°C and 350°C). The spectra were recorded at room temperature. All the spectra were scaled according to the sample weight. Brønsted and Lewis acidity of the samples compared are given in arbitrary units, according to the intensity of the bands assigned to the pyridine interacting with the Brønsted and Lewis acid sites of the zeolites (1550 and 1450 cm⁻¹, respectively). These acidic properties are listed in Table 2 provide *infra.*

**Table 2: Acidic properties of samples**

| | **Brønsted Acidity (u.a.)** | | | | **Lewis Acidity (u.a.)** | | |
|---|---|---|---|---|---|---|---|
| **Sample** | **B150** | **B250** | **B350** | **B350/B150** | **L150** | **L250** | **L350** |
| SSZ-33 | 375 | 355 | 311 | 0.83 | 206 | 210 | 168 |
| Re/SSZ-33 Example 4 (comparative) | 345 | 328 | 247 | 0.72 | 263 | 251 | 240 |
| ZSM-5 (CBV3024E) | 439 | 395 | 337 | 0.77 | 51 | 36 | 32 |
| Re/ZSM-5 Example 6 (comparative) | 411 | 336 | 293 | 0.71 | 94 | 68 | 61 |
| AC-140 Example 5 (comparative) | 110 | 72 | 45 | 0.45 | 252 | 207 | 148 |

| | | | | | | | |
|---|---|---|---|---|---|---|---|
| *Normalized to zeolite content as AC-140 comprises non-zeolite content. | | | | | | | |

Table 2 illustrates the comparative acidic properties of SSZ-33 and ZSM-5, individually as well as those of the commercially available AC-140 multizeolite catalyst. It is noted that the AC-140 has a markedly lesser acidity than the SSZ-33 and ZSM-5 individually despite the AC-140 acidity referencing to the actual zeolite content. Additionally, Brønsted acid site density of the SSZ-33 is less than that of ZSM-5, although their acid strength distribution is comparable. It is further noted, the number of Lewis acid sites is significantly greater for the SSZ-33. The difference in Brønsted acid site density and number of Lewis acid sites may be attributed to the presence of extraframework Aluminum (EFAL) in the SSZ-33 with part of the EFAL generating more Lewis acid sites while neutralizing bridging hydroxyl groups, thereby decreasing the Brønsted acid density. An increase in the Lewis acidity is also noted for all the zeolites upon addition of the rhenium.

As stated previously, the present composite zeolite catalyst represent a dealkylation and transalkylation catalyst suitable for converting C₉₊ alkyl aromatic hydrocarbons to a product stream comprising benzene, toluene, and xylenes, particularly to commercially valuable xylenes. The feed stream to the conversion process generally comprises alkylaromatic hydrocarbons in the carbon number range C₉ to C₁₁₊ that may include, for example, such hydrocarbons as propylbenzenes, methylethylbenzenes, tetramethylbenzenes, ethyldimethylbenzenes, diethylbenzenes, methylpropylbenzenes, and mixtures thereof. For purposes of testing and quantifying the examples and comparative examples a simulated heavy reformate feed was generated. The simulated heavy reformate feed comprised 30 wt. % *para*-methylethylbenzene (*p*-MEB) and 70 wt. % 1,2,4-trimethylbenzene (1,2,4-TMB).

Catalytic tests for conversion of the simulated heavy reformate feed were performed in a reaction system comprising sixteen (16) continuous fixed-bed parallel microreactors. Each reactor was capable of being fed independently with the desired flow of the simulated heavy reformate feed and H₂ making it possible to operate in a wide range of contact times and hydrogen/hydrocarbon molar ratios. The simultaneous catalytic experiments were carried out under the following conditions: 20 bar total pressure, a hydrogen/hydrocarbon molar ratio of 8.5, a reaction time of 16 hours per temperature, and a weight hourly space velocity (WHSV) of 10 inverse hours (h⁻¹). After the testing at each temperature, the zeolitic catalysts were kept at that temperature and under H₂ atmosphere for 10 additional hours. Each zeolitic catalyst sample was prepared to a particle size of 0.2 to 0.4 millimeters (mm). The tested zeolitic samples included Example 1 (60:40 weight ratio SSZ-33:ZSM-5 with 0.3 wt. % rhenium) synthesized in accordance with the present disclosure, Example 2 (40:60 weight ratio SSZ-33:ZSM-5 with 0.3 wt. % rhenium) synthesized in accordance with the present disclosure, Example 3 (80:20 weight ratio SSZ-33:ZSM-5 with 0.3 wt. % rhenium) synthesized in accordance with the present disclosure, Example 4 (comparative) (SSZ-33 with 0.3 wt. % rhenium), and Example 5 (comparative) (commercially available AC-140). Each fixed-bed microreactor reactor was prepared with 125 mg of the zeolitic catalyst sample and diluted with silicon carbide (SiC) to a total bed volume of 2.0 ml for testing. The experiments were performed on the same zeolite weight basis so the non-zeolite components of the AC-140 were excluded from calculation of the 125 mg. Prior to commencing the catalytic test, the catalyst was reduced in situ at 450°C for 1 hour (h) under H₂ flow (50 ml/min) at atmospheric pressure. Four consecutive reactions phases were completed at temperatures of 350°C, 375°C, 400°C, and a return to 350°C.

The reaction products from each of the fixed-bed microreactors were analyzed by a Bruker 450-GC on-line gas chromatography using two independent channels. Nitrogen (N₂) as an internal standard, H₂, methane, and ethane were analyzed in a first channel equipped with a thermal conductivity detector (TCD) and three columns. The three columns were a Hayesep N pre-column (0.6 meter (m) length) (Hayes Separations, Inc.), a Hayesep Q (1.6 m length) (Hayes Separations, Inc.), and a 13X molecular sieve (1.2 m length). In a second channel the C₁-C₄ hydrocarbons were first separated from the aromatics in a CP-Wax capillary column (5.0 m length and 0.32 millimeter (mm) inner diameter) (Cole-Parmer). Subsequently, the C₁-C₄ gases were separated in a column with CP-PoraBOND Q (25 m length and 0.32 mm inner diameter) (Cole-Parmer) and detected in a flame ionization detector (FID). Separation of the aromatics was completed in a second CP-Wax (1.0 m length and 0.32 mm inner diameter) connected to a second FID.

With reference to FIGS. 1, 2, and 3, the MEB conversion (dealkylation), TMB conversion (transalkylation), and overall conversion (MEB+TMB) are illustrated for each of Example 1, Example 2, Example 3 Example 4 (comparative), and Example 5 (comparative). It is noted that Examples 1, 2 and 3 (SSZ-33 and commercial ZSM-5, each with 0.3 wt. % rhenium) and Example 4 (comparative) (SSZ-33 with 0.3 wt. % rhenium) did not exhibit deactivation during the testing procedure. Conversely, Example 5 (comparative) (AC-140) exhibited substantial deactivation This phenomenon is indicated by the conversion percentage for the initial 350°C stage at the beginning of each test and the final 350°C stage at the conclusion of each test being similar for Examples 1, 2, 3 and Example 4 (comparative) and with a marked difference for Example 5 (comparative).

The lack of deactivation observed for the composite zeolite catalyst (Examples 1, 2, and 3) is believed to be due to the greater catalytic efficiency, which reduces the formation of heavy alkylaromatics. The specific microporous structure of SSZ-33, combining medium (10-ring) and large pore (12-ring) channels confers the composite zeolite catalyst high overall activity and high selectivity to the transalkylation of the TMB present in the feed with the toluene formed in-situ on the commercial ZSM-5.

With reference once again to FIGS. 1, 2, and 3, the overall conversion (MEB+TMB) with Examples 1, 2, and 3 is maintained equal to Example 4 (comparative) and greater to Example 5 (comparative). Mixing the SSZ-33 zeolite of Example 4 (comparative) with 0.3 wt. % rhenium infused commercially available ZSM-5 decreased the transalkylation activity as evidenced by the lesser TMB conversion of Examples 1, 2, and 3 than Example 4 (comparative). However, mixing the SSZ-33 zeolite of Example 4 (comparative) with 0.3 wt. % rhenium infused commercially available ZSM-5 increased the dealkylation activity as evidenced by the greater MEB conversion of Examples 1, 2, and 3 than Example 4 (comparative) to result in an overall conversion substantially the same for Examples 1, 2, and 3 and Example 4 (comparative).

With reference to FIGS. 4, 5, 6, 7, 8, 9 and 10, the xylenes yield, A₁₀ yield, A₁₀₊ yield, light hydrocarbon yield, toluene yield, ethylbenzene yield, and benzene yield are respectively illustrated for each of Example 1, Example 2, Example 3, Example 4 (comparative), and Example 5 (comparative) versus time on stream (TOS). It is noted that Examples 1, 2, and 3 favors the xylenes production as compared to both Example 4 (comparative) and Example 5 (comparative) as shown in FIG. 4. The greater selectivity to xylenes is believed to be a consequence of the lesser production of undesirable A₁₀₊ aromatics. As indicated in FIG. 6, Examples 1, 2, and 3 demonstrated the lowest yield of A₁₀₊ aromatics. Similarly, Examples 1, 2, and 3 also demonstrate greater yields of other desirable products including toluene as shown in FIG. 8, benzene as shown in FIG. 10, and light hydrocarbons as shown in FIG. 7. It is also noted that production of undesirable ethylbenzene with Examples 1, 2, and 3 is lesser than for Example 4 (comparative) and Example 5 (comparative) as illustrated in FIG. 9.

The results generated from testing the samples with the simulated heavy reformate provided information regarding the relative activity of the different catalyst compositions and their stability towards deactivation with an extended TOS. The catalysts were also tested under conditions closer to industrial conditions which would be observed for conversion of heavy reformate to xylenes and other light hydrocarbon products. To more accurately reflect industrial conditions a supply of actual industrial heavy reformate with known composition was utilized. Table 3 delineates the composition of the industrial heavy reformate used for testing and Table 4 provides the relative ratios of various components.

**Table 3: Industrial Heavy Reformate Composition**

| **Component** | | | **Mass %** |
|---|---|---|---|
| **Hydrocarbon Type** | **Hydrocarbon Sub-Type** | | |
| A₈ | Total | | 3.94 |
| | Ethylbenzene | | 0.03 |
| | *p*-xylene | | 0.15 |
| | *m*-xylene | | 0.38 |
| | *o*-xylene | | 3.38 |
| A₉ | Total | | 82.75 |
| | Isopropylbenzene | Total | 0.43 |
| | n-propylbenzene | Total | 2.07 |
| | Methylethylbenzene (MEB) | Total | 19.62 |
| | | *m*- and *p*-MEB | 15.33 |
| | | *o*-MEB | 4.29 |
| | Trimethylbenzene (TMB) | Total | 60.63 |
| | | 1,3,5-TMB | 11.69 |
| | | 1,2,4-TMB | 40.81 |
| | | 1,2,3-TMB | 8.13 |
| A₁₀₊ | Total | | 13.33 |

**Table 4: Industrial Heavy Reformate Composition**

| **Ratio** | | |
|---|---|---|
| **A₈** | | |
| | Ethylbenzene : Total A₈ | 0.0076 |
| **Ratio** | | |
| | *p*-xylene : Total A₈ | 0.038 |
| | *m*-xylene : Total A₈ | 0.096 |
| | *o*-xylene : Total A₈ | 0.858 |

| **A₉** | | |
|---|---|---|
| | Isopropylbenzene : Total A₉ | 0.0052 |
| | n-propylbenzene : Total A₉ | 0.025 |
| | Total Methylethylbenzene (MEB) : Total A₉ | 0.237 |
| | *m*- and *p*-MEB : Total Ag | 0.185 |
| | *o*-MEB : Total A₉ | 0.052 |
| | *m*- and *p*-MEB : Total MEB | 0.781 |
| | *o*-MEB : Total MEB | 0.219 |
| | Total Trimethylbenzene (TMB) : Total A₉ | 0.733 |
| | 1,3,5-TMB : Total A₉ | 0.141 |
| | 1,2,4-TMB : Total A₉ | 0.493 |
| | 1,2,3-TMB : Total A₉ | 0.098 |
| | 1,3,5-TMB : Total TMB | 0.193 |
| | 1,2,4-TMB : Total TMB | 0.673 |
| | 1,2,3-TMB : Total TMB | 0.124 |
| | Total A₉ : Total A₁₀₊ | 6.21 |

Catalytic test for conversion of the industrial heavy reformate feed were performed in a fixed-bed stainless-steel tubular reactor. The reactor had a 10.5 mm internal diameter and a 20 centimeter (cm) length. The catalytic experiments in the fixed-bed tubular reactor were carried out under the following conditions: 20 bar total pressure, a hydrogen/hydrocarbon molar ratio of 4:1, a reaction time of 5 to 7 hours at each reaction temperature, and a weight hourly space velocity (WHSV) of 10 h⁻¹. The reactor was charged with 0.75 grams (g) of catalyst with a particle size of 0.2 to 0.4 mm for each test. The tested zeolitic samples included Example 1 (60:40 weight ratio SSZ-33:ZSM-5 with 0.3 wt. % rhenium) synthesized in accordance with the present disclosure, Example 4 (comparative) (SSZ-33 with 0.3 wt. % rhenium), and Example 5 (comparative) (commercially available AC-140). Each catalyst was diluted with SiC to bring the total volume up to a total bed volume of 5.0 ml. For the commercial AC-140 sample (Example 5 (comparative)), the amount of catalyst added was adjusted according to the zeolite content in order to have 0.75 g of zeolite. Gaseous compounds (H₂, N₂) were fed into the system by mass flow meters via a vaporizer. Nitrogen was also fed into the system as an internal reference. The industrial heavy reformate was fed by means of a high performance liquid chromatography (HPLC) pump to the vaporizer. The vaporizer was operated at 300°C and provided a steady and non-pulsing flow of reactants to the reactor. Prior to commencing the catalytic test, the catalyst was reduced *in situ* at 450°C for 1 h under H₂ flow (50 milliliters per minute (ml/min)) at atmospheric pressure. For the catalytic testing, four consecutive reaction phases were completed at temperatures of 350°C (7 hour reaction), 375°C (5 hour reaction), 400°C (5 hour reaction), and a return to 350°C (5 hour reaction).

During reaction, the effluent stream was analyzed on-line at intervals of 32 minutes (min) in a Scion 456 Gas Chromatograph equipped with two detection channels. Nitrogen (N₂) as an internal standard, H₂, methane, and ethane were analyzed in a first channel equipped with a TCD and three columns. The three columns were a Hayesep N pre-column (0.6 meter (m) length) (Hayes Separations, Inc.), a Hayesep Q (1.6 m length) (Hayes Separations, Inc.), and a 13X molecular sieve (1.2 m length). In a second channel the C₁-C₄ hydrocarbons were first separated from the aromatics in a CP-Wax capillary column (5.0 m length and 0.32 mm inner diameter) (Cole-Parmer). Subsequently, the C₁-C₄ gases were separated in a column with CP-PoraBOND Q (25 m length and 0.32 mm inner diameter) (Cole-Parmer) and detected in a flame ionization detector (FID). Separation of the aromatics was completed in a second CP-Wax (39.5 m length and 0.32 mm inner diameter) connected to a second FID.

With reference to FIGS. 11, 12 and 13, the TMB conversion (transalkylation), MEB conversion (dealkylation), and overall conversion (MEB+TMB) are illustrated for each of Example 1, Example 4 (comparative), and Example 5 (comparative). It is noted that Example 1 indicated greater overall conversion than either of Example 4 (comparative) or Example 5 (comparative). The greater overall conversion may be attributed to greater MEB conversion for Example 1 as compared to Example 4 (comparative), and similar to Example 5 (comparative), and an increased TMB conversion of Example 1 when compared to Example 4 (comparative) and Example 5 (comparative). The individual TMB conversion percentages and MEB conversion percentages are provided in Table 5.

**Table 5: TMB, MEB, and Overall Conversion**

| **Catalyst** | **Temperature** | **MEB Conversion (%)** | **TMB Conversion (%)** | **Overall Conversion (%)** |
|---|---|---|---|---|
| Example 1 | 350°C | 82.58 | 58.01 | 64.61 |
| | 375°C | 91.09 | 62.12 | 69.90 |
| | 400°C | 95.21 | 63.65 | 72.12 |
| | 350°C (Return) | 75.31 | 50.30 | 57.02 |
| Example 4 (comparative) | 350°C | 76.34 | 40.86 | 50.23 |
| | 375°C | 80.18 | 40.40 | 50.91 |
| | 400°C | 88.46 | 45.25 | 56.67 |
| | 350°C (Return) | 74.66 | 46.31 | 53.80 |
| Example 5 (comparative) | 350°C | 78.58 | 35.51 | 46.89 |
| | 375°C | 87.01 | 32.79 | 47.11 |
| | 400°C | 92.95 | 35.19 | 50.45 |
| | 350°C (Return) | 65.48 | 12.66 | 26.62 |

With reference to FIGS. 14, 15, 16, 17, 18, 19, and 20, the xylenes yield, A₁₀ yield, A₁₀₊ yield, light hydrocarbon yield, toluene yield, ethylbenzene yield, and benzene yield are respectively illustrated for each of Example 1, Example 4 (comparative), and Example 5 (comparative). It is noted, with reference to FIG. 14, that Example 1 favors the xylenes production as compared to Example 4 (comparative) and Example 5 (comparative). As xylenes are the desirable product, the increased yield of xylenes with the composite zeolite catalyst comprising SSZ-33 and ZSM-5 is a positive result. The greater selectivity to xylenes is believed a consequence of the lesser production of undesirable A₁₀₊ aromatics. As indicated in FIG. 16, Example 1 demonstrated the lowest yield of A₁₀₊ aromatics. Similarly, Example 1 also demonstrates greater yields of other products including toluene as shown in FIG. 18, benzene as shown in FIG. 20, and light hydrocarbons as show in FIG. 17. The numerical values of the yield as a wt. % for each catalyst are provided in Table 6. This improvement in xylenes and other light hydrocarbon production and concurrent reduction in A₁₀₊ fraction illustrates the benefit of the methods of the present disclosure where SSZ-33 and ZSM-5 are in proximate contact in a single reactor during heavy reformate feed conversion. An additional advantage is that the conversion may be completed in a single reactor, thereby reducing production complexity.

**Table 6: Product Yields**

| **Catalyst** | **Temperat ure** | **Xylenes Yield (wt.%)** | **A₁₀ Yield (wt.%)** | **A₁₀₊ Yield (wt.%)** | **Light HC Yield (wt.%)** | **Toluene Yield (wt.%)** | **Ethylbenzene Yield (wt.%)** | **Benzene Yield (wt.%)** |
|---|---|---|---|---|---|---|---|---|
| Example 1 | 350°C | 35.16 | 3.67 | 5.03 | 9.63 | 15.27 | 1.03 | 1.84 |
| | 375°C | 37.63 | 2.10 | 4.34 | 11.17 | 17.76 | 0.57 | 2.31 |
| | 400°C | 37.17 | 1.05 | 4.33 | 13.81 | 18.51 | 0.28 | 2.51 |
| | 350°C (Return) | 28.40 | 3.73 | 4.43 | 7.22 | 17.50 | 1.89 | 2.33 |
| Example 4 (comparative) | 350°C | 21.74 | 8.06 | 19.66 | 3.74 | 6.56 | 0.78 | 0.60 |
| | 375°C | 24.58 | 6.70 | 17.27 | 4.52 | 7.26 | 0.65 | 0.68 |
| | 400°C | 28.31 | 4.07 | 16.38 | 6.73 | 9.21 | 0.40 | 0.93 |
| | 350°C (Return) | 13.47 | 6.71 | 35.94 | 1.88 | 4.62 | 0.79 | 0.48 |
| Example 5 (comparative) | 350°C | 20.94 | 6.91 | 15.08 | 4.96 | 8.34 | 0.90 | 1.08 |
| | 375°C | 23.28 | 4.20 | 10.49 | 6.16 | 11.83 | 0.71 | 1.69 |
| | 400°C | 23.96 | 3.30 | 11.59 | 7.91 | 12.01 | 0.34 | 1.91 |
| | 350°C (Return) | 12.36 | 5.13 | 9.06 | 4.58 | 9.00 | 1.13 | 1.33 |

It should not be understood the various aspects of the composite zeolite catalyst, the method of making the same, the method of making xylene using the same, and a system for making xylene using the same are described and such aspects may be utilized in conjunction with various other aspects.

It should be apparent to those skilled in the art that various modifications and variations can be made to the described embodiments without departing from the spirit and scope of the claimed subject matter. Thus, it is intended that the specification cover the modifications and variations of the various described embodiments provided such modification and variations come within the scope of the appended claims and their equivalents.

Throughout this disclosure ranges are provided. It is envisioned that each discrete value encompassed by the ranges are also included. Additionally, the ranges which may be formed by each discrete value encompassed by the explicitly disclosed ranges are equally envisioned.

## Claims

1. A method of making BTX compounds including benzene, toluene, and xylene, the method comprising:
feeding heavy reformate to a reactor, the reactor containing a composite zeolite catalyst comprising a mixture of SSZ-33 and ZSM-5, where the SSZ-33, the ZSM-5, or both comprise one or more impregnated metals; and
producing the BTX compounds by simultaneously performing transalkylation and dealkylation of the heavy reformate in the reactor, where the composite zeolite catalyst is able to simultaneously catalyze both the transalkylation and dealkylation reactions.

2. The method of claim 1, where the heavy reformate comprises at least 15 wt. % methylethylbenzene (MEB) and at least 50 wt. % trimethylbenzene (TMB).

3. The method of any of claims 1 or 2, where the one or more impregnated metals are selected from the group consisting of molybdenum, chromium, platinum, nickel, tungsten, palladium, ruthenium, gold, rhenium, rhodium, or combinations thereof and their respective oxides.

4. The method of any of claims 1 to 3, where the one or more impregnated metals comprise rhenium.

5. The method of any of claims 1 to 4, where the SSZ-33, the ZSM-5, or both the SSZ-33 and ZSM-5 comprise 0.01 wt. % to 20 wt. % of the one or more impregnated metals.

6. The method of any of claims 1 to 5, where the composite zeolite catalyst comprises a mixture of SSZ-33 and ZSM-5 in a 50:50 to 90:10 weight ratio.

7. The method of any of claims 1 to 5, where the composite zeolite catalyst comprises a mixture of SSZ-33 and ZSM-5 in a 50:50 to 80:20 weight ratio.

8. The method of any of claims 1 to 7, where the SSZ-33 is impregnated with 0.25 to 0.55 wt. % rhenium.

9. The method of any of claims 1 to 8, where the ZSM-5 is impregnated with 0.25 to 0.55 wt. % rhenium.

10. The method of any of claims 1 to 9, where the SSZ-33 is an Aluminum-SSZ-33.

11. The method of any of claims 1 to 10, where the reactor is operated at 350°C to 400°C.
